(19)

Europäisches Patentamt

European Patent Office

Office européen des brevets

(11) **EP 2 341 388 A1**

(12) **EUROPEAN PATENT APPLICATION**
published in accordance with Art. 153(4) EPC

(43) Date of publication:
06.07.2011 Bulletin 2011/27

(21) Application number: **09820561.0**

(22) Date of filing: **13.10.2009**

(51) Int Cl.:
*G02C 13/00* (2006.01)　　　*G02C 7/06* (2006.01)

(86) International application number:
**PCT/JP2009/067693**

(87) International publication number:
**WO 2010/044383 (22.04.2010 Gazette 2010/16)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **17.10.2008 JP 2008269053**
**17.10.2008 JP 2008269054**

(71) Applicant: **Hoya Corporation**
**Tokyo 161-8525 (JP)**

(72) Inventors:
• **QI Hua**
**Tokyo 161-8525 (JP)**
• **TANAKA Ikuka**
**Tokyo 161-8525 (JP)**
• **TAKETO Kenji**
**Tokyo 161-8525 (JP)**

(74) Representative: **Betten & Resch**
**Patentanwälte**
**Theatinerstrasse 8**
**80333 München (DE)**

(54) **VISUAL FIELD IMAGE DISPLAY DEVICE FOR EYEGLASSES AND METHOD FOR DISPLAYING VISUAL FIELD IMAGE FOR EYEGLASSES**

(57)　　　The present invention is made to provide a visual field image display device for spectacles capable of displaying a retinal image by simulation corresponding to change even in visual line direction.

A visual field image display device for spectacles adapted to display a retinal image seen when wearing a progressive-addition lens by simulation includes: an image processing section adapted to create the retinal image by performing a processing of adding blur and distortion to data of an original image in a visual field corresponding to the direction of a visual line of an eye of a person in a state where the person wears the progressive-addition lens, wherein the blur and distortion correspond to a passing point of the visual line on the progressive-addition lens; and a display section adapted to display the retinal image created by the image processing section.

FIG. 1

EP 2 341 388 A1

## Description

### Technical Field

The present invention relates to a visual field image display device for spectacles adapted to display a retinal image seen when wearing a spectacle lens by simulation, and a method for displaying visual field image for spectacles.

### Background Art

Conventionally, in a spectacles store, a spectacle wearer wears sample lenses to confirm the vision, and selects lenses and frame to place an order.

However, the number of kinds of the sample lenses possible to be prepared by the spectacles store is limited. Since there are so many kinds of lenses particularly in the case where the lens is a progressive-addition lens, there is no guarantee that there is a sample lens suited to the spectacle wearer among the sample lenses. For this reason, the spectacle wearer can not know what vision is to be seen through the ordered lens until he (or she) actually wears the ordered lens after the lens is completed.

To solve such a problem, it is proposed to display a vision to be seen through a lens not covered by the sample lenses by displaying a retinal image seen when wearing spectacles by simulation (see, for example, Patent Document 1). In a progressive-addition lens, since a distance portion and a near portion, which have different powers from each other, are smoothly connected to each other, distortion is generated. Therefore there will be image fluctuation when direction of the face of the spectacle wearer changes. By displaying the retinal image seen when wearing spectacles by simulation, it becomes possible to easily show the image fluctuation.

In the configuration described in Patent Document 1, the image range is changed according to the change of the direction of the face of the spectacle wearer, and an image processing with respect to the distortion is performed on the image in that range.

[Prior art documents]

[Patent documents]

[Patent Document 1] Specification of Japanese Patent No. 3893760

### Disclosure of the Invention

### Problems to be Solved by the Invention

In the configuration described in the aforesaid patent document, since the image processing with respect to the distortion is simply performed on the image in the range of the visual field, calculation is relatively simple, and it is possible to easily simulate the retinal image seen when wearing a progressive-addition lens.

However, it is not possible to precisely simulate an image seen when actually wearing the progressive-addition lens.

To be specific, when actually using a progressive-addition lens, the vision is little changed by only changing the direction of the face. Typically the progressive-addition lens wearer changes the angle of his (or her) face to change the direction of the visual field just for purpose of reducing the image fluctuation and distortion.

Further, changeover between the near portion and the distance portion is mainly achieved by moving visual line by cycloduction.

Thus, although the vision (the direction of the visual field) changes when the spectacle wearer changes the angle of his (or her) face, the changeover between the near portion and the distance portion is typically achieved by the change of the visual line direction (i.e., the angle of the cycloduction).

With the configuration described in the aforesaid patent document, for example, if the spectacle wearer lowers his (or her) face with his (or her) visual line fixed at the distance power position of the progressive-addition lens, the simulation will be performed based on the judgment that the spectacle wearer is looking at the near power position.

Thus, the magnification and distortion of the image will become different from those corresponding to the fact that the visual line is fixed at the distance power position, and therefore it is not possible to precisely perform simulation.

Further, similar problem will arise if the spectacle wearer raises his (or her) face with his (or her) visual line fixed at the near power position.

Since the difference of the magnification in the lateral direction is not as large as that in the up-down direction, it is, on some level, possible to simulate the peripheral vision by moving the face in the lateral direction.

However, it is not possible to simulate the peripheral vision portion and the principal gazing vision portion by moving the face in the up-down direction.

To solve the aforesaid problems, it is an object of the present invention to provide a visual field image display device for spectacles capable of displaying a retinal image by simulation corresponding to the change even in visual line direction. Further, it is another object of the present invention to provide a method for displaying visual field image for spectacles capable of displaying a retinal image close to the actual retinal image seen when actually wearing spectacles by simulation.

**Means for Solving the Problems**

A visual field image display device for spectacles according to an aspect of the present invention is adapted to display a retinal image seen when wearing a progressive-addition lens by simulation. The device includes: an image processing section adapted to create the retinal image by performing a processing of adding blur and distortion to data of an original image in a visual field corresponding to the direction of a visual line of an eye of a person in a state where the person wears the progressive-addition lens, wherein the blur and distortion correspond to a passing point of the visual line on the progressive-addition lens; and a display section adapted to display the retinal image created by the image processing section.

The aforesaid visual field image display device for spectacles can be configured so that the distortion is calculated by using brightness information of the original image, the brightness information being specified at a point in a visual field on an object side corresponding to a pixel sampled in a visual field on an image side.

Further, the aforesaid visual field image display device for spectacles can be configured so that the blur is calculated based on a continuous function which expresses a light distribution of light-rays from an object, the light-rays spreading with an image point as the center.

Further, in addition to the aforesaid configuration, the aforesaid visual field image display device for spectacles can be configured so that the processing of adding blur is performed by distributing the brightness of each pixel of the display section to peripheral pixels base on the continuous function, and recalculating the brightness of all pixels of the image.

Further, in addition to the aforesaid configuration, the aforesaid visual field image display device for spectacles can be configured so that the processing of adding blur is performed by using a normal distribution function and parameters expressed as the following equation (1) and including an equiprobability ellipse which expresses the level of the spreading, the distortion and the blur of the image point:

$$f(\mu, \nu) = \frac{1}{2\pi\sigma_\mu\sigma_\nu\sqrt{1-\rho^2}} \exp\left(-\frac{c^2}{2}\right) \quad \text{------ (1)}$$

(here, $\mu$, $\nu$ are respectively the deflection angles of the principal light-ray from the coordinate axis direction, and $\sigma_\mu$, $\sigma_\nu$, $\rho$ are parameters of the normal distribution, wherein $\sigma_\mu > 0$, $\sigma_\nu > 0$, $-1 < \rho < 1$)

Further, in addition to the aforesaid configuration, the aforesaid visual field image display device for spectacles can be configured so that the parameters are calculated based on a one-dimensional nested structure.

The aforesaid visual field image display device for spectacles can be configured so that the direction of the visual line is identified by a visual line tracking device and a gyro sensor.

Further, in addition to the aforesaid configuration, the aforesaid visual field image display device for spectacles can be configured so that the display section includes a head mounted display, and the visual line tracking device and the gyro sensor are mounted on the head mounted display.

A method for displaying visual field image for spectacles according to another aspect of the present invention is adapted to display a retinal image seen when wearing a progressive-addition lens by simulation. The method includes: calculating the retinal image to be displayed at each point of a point group spirally arranged within a visual field.

The aforesaid visual field image display device for spectacles can be configured so that the spiral arrangement includes at least two spirals.

Further, the aforesaid visual field image display device for spectacles can be configured so that the spiral arrangement includes six spirals.

**Advantages of the Invention**

With the visual field image display device for spectacles according to the present invention, in the image processing section, the retinal image is created by performing the processing of adding the blur and distortion to data of the original image in the visual field corresponding to the direction of a visual line of an eye of a person, wherein the blur and distortion correspond to a passing point of the visual line on the progressive-addition lens.

Because of this feature, it is possible to create a retinal image in which a processing of adding blur and distortion is performed while the change of the visual line passing point on the lens caused not only by the change of the direction of face but also by the change of the visual line direction is reflected. Thus, it is easy to accurately simulate the peripheral vision portion and the principal gazing vision portion.

Thus, with the visual field image display device for spectacles of the present invention, it is possible to achieve a visual field image display device for spectacles capable of displaying a retinal image close to an actual vision corresponding to even the change of the visual line direction.

According to the method for displaying visual field image for spectacles of the present invention, the retinal image to be displayed at each point of a point group spirally arranged within a visual field is calculated.

By using a spirally arranged point group, it is possible to arrange more point groups in the visual field compared with, for example, the case where a vertically and horizontally arranged point group is used, so that the area within the visual field can be more efficiently used to calculate a retinal image close to the actually seen retinal image.

Thus, with the method for displaying visual field image for spectacles of the present invention, it is possible to display a retinal image close to the retinal image seen when actually wearing spectacles by simulation.

**Brief Description of Drawings**

FIG. 1 is a block diagram showing a schematic configuration of a visual field image display device for spectacles (a display system) according to an embodiment of the present invention;

FIG. 2 is a view showing an example of a three-dimensional CG model used in the system shown in FIG. 1;

FIG. 3 is a flowchart for explaining the steps performed by the device shown in FIG. 1 until a simulation image is displayed;

FIG. 4 shows a coordinate system used in the simulation according to the aforesaid embodiment of the present invention;

FIG. 5 is a view for explaining distortion of light-rays caused by the refraction of a lens;

FIG. 6 is a view for explaining light-ray tracing for obtaining PSF;

FIG. 7A and FIG. 7B are views each showing a method of dividing an entrance pupil;

FIG. 8 is a view showing correspondence between the image position on the retina and the incidence angle; and

FIG. 9 is a view showing an equiprobability ellipse.

**Best Modes for Carrying Out the Invention**

The best mode for carrying out the present invention (referred to as "embodiment" hereinafter) will be described below.

The description will be made in the following order.

1. Description of configuration of device/system according to an embodiment of the present invention
2. Description of theory and method of moving image simulation
2-1. Purpose of simulation
2-2. Coordinate system used in simulation
2-3. Description of distortion of lens
2-3. Description of blur of lens
2-5. Synthesis of simulation image
2-6. Coordinate transformation in a case where central visual line of visual field is designated
2-7. Spline interpolation approximation of light-ray data
2-8. Simplification of PSF
2-9. Speeding up multi-dimensional spline interpolation calculation
2-10. Summary

<1. Description of configuration of device/system according to an embodiment of the present invention>

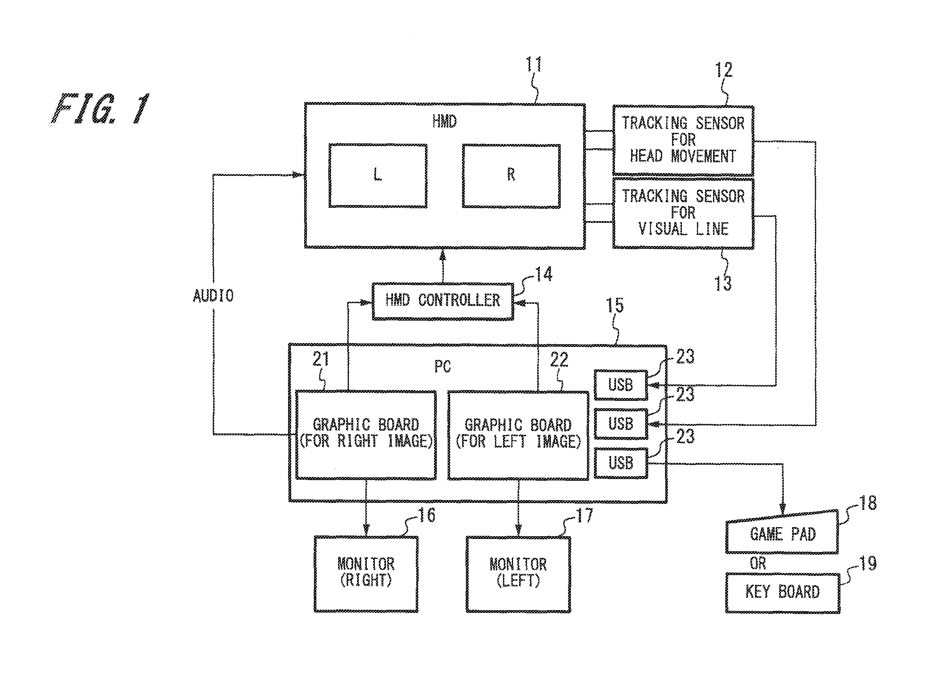

A block diagram of a schematic configuration of a visual field image display device for spectacles (a display system), as an embodiment of the present invention, is shown in FIG. 1.

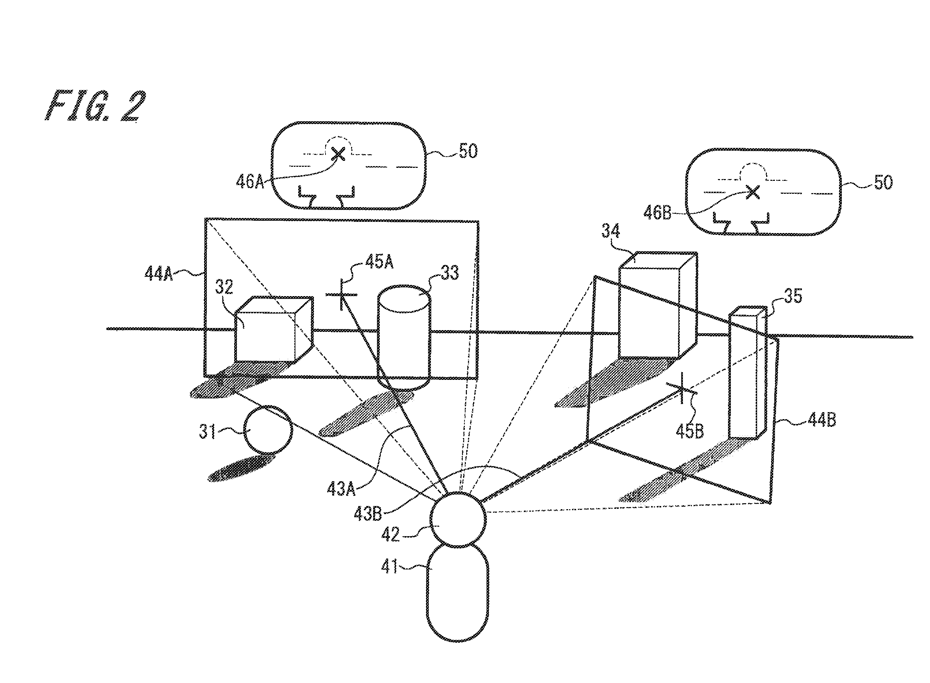

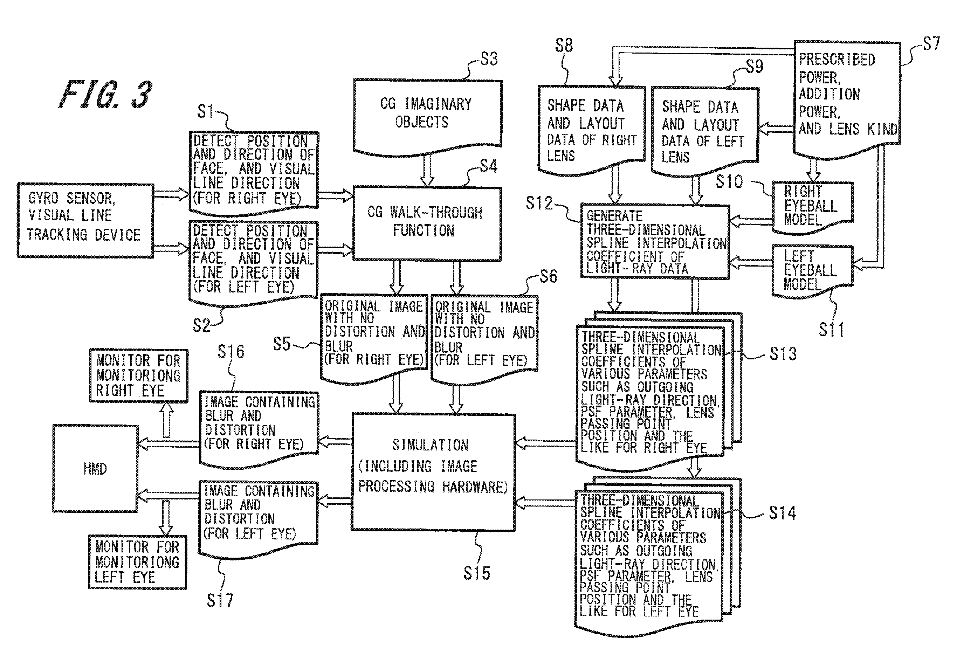

The system shown in FIG. 1 includes a HMD (head mounted display) 11, a PC (personal computer) 15, two monitors 16, 17, and a game pad 18 or a keyboard 19 (as input device).
A tracking sensor for head movement (such as a gyro sensor or the like) 12 and a tracking sensor for visual line (such as a visual line tracking device) 13 are mounted on the HMD 11.
A graphic board 21 for image for the right eye, a graphic board 22 for image for the left eye, and a USB 23 are provided in the PC 15. Further, a HMD controller 14 is connected between the HMD 11 and the PC 15. Due to the provision of the graphic boards 21, 22, the PC 15 functions as an image processing section of the visual field image display device for spectacles of the present invention.
A purpose of the present invention is to allow the spectacle wearer to experience a vision seen through a progressive-addition lens in terms of "image fluctuation (i.e., distortion)" and "blur".
To serve this purpose, an image processing effect of the "distortion" and "blur" is added to a three-dimensional CG (Computer Graphics) movie (which is the object to be viewed) by performing real-time calculation, so that the vision seen through the progressive-addition lens is recreated.
In the system shown in FIG. 1, a stereoscopic vision can be displayed by preparing an image for the right eye and an image for the left eye.
Further, the recreated visual field is displayed in a manner in which the visual field in the three-dimensional CG model is tracked by the gyro sensor (i.e., the tracking sensor for head movement 12) and the visual line tracking device (i.e., the tracking sensor for visual line 13) mounted on the HMD 11 according to the movement of the visual line of the viewer.
In the PC 15, an image processing functioning as a "distortion filter" and a "blur filter" is performed.
The "distortion filter" is a mechanism for returning the value of an output coordinate (T', C') on the image side based on a B spline light-ray database, with respect to each pixel of an original image.
The "blur filter" is a mechanism for returning the values of the size ($\sigma_\mu$, $\sigma_\nu$) and density ($\rho$ of the blur texture based on the B spline light-ray database, with respect to each pixel of the original image.
All pixels of the original image are functioned as the input, and a blur texture image returned through the "blur filter" is added (i.e., overpainted) on the coordinate position of the outputted image returned through the "distortion filter", so that a visible image (the output) is generated.
An example of the three-dimensional CG model used in the system of FIG. 1 is shown in FIG. 2.
Five objects 31, 32, 33, 34, 35 respectively having shapes of a ball, a cylinder, a rectangular parallelepiped, a cube and the like are arranged in a space. When viewing from a spectacle wearer 41, the distances respectively from the object 31, 32, 33, 34, 35 are different. The distance from the ball-shaped object 31 is relatively smaller, and the distances from the object 32 and object 34 are relatively larger.
Further, in FIG. 2, in addition to the arrangement of the three-dimensional CG model, visual line passing points 46A, 46B on a spectacle lens 50 are shown in principal visual lines 43A, 43B in two directions.
The fact that the positions of the visual line passing points on the spectacle lens 50 change according to the position of the visual field will be described below with reference to FIG. 2.
First, a condition is considered in which the spectacle wearer 41 views the object 32 and the object 33, and sees a visual vision 44A. At this time, although not shown, the head 42 of the spectacle wearer 41 is directed to the visual vision 44A. Further, the principal visual line 43A is directed to the center of a visual field 45A of the visual vision 44A. Here, since the distance of the visual vision 44A from the spectacle wearer 41 is relatively larger, the position of the visual line passing point 46A on the spectacle lens 50 is slightly higher than the center portion.
Next, a condition is considered in which the spectacle wearer 41 views the object 34 and the object 35, and sees a visual vision 44B. At this time, although not shown, the head 42 of the spectacle wearer 41 is directed to the visual vision 44B. Further, the principal visual line 43B is directed to the center of a visual field 45B of the visual vision 44B. Here, since the distance of the visual vision 44B from the spectacle wearer 41 is smaller than the distance of the visual vision 44A, the position of the visual line passing point 46B on the spectacle lens 50 is located near the center portion of the spectacle lens 50, which is lower than the aforesaid position of the visual line passing point 46A.
Thus, due to the change of the visual visions 44A, 44B, not only the direction of the head 42 of the spectacle wearer 41 is changed, but also the visual line passing points 46A, 46B on the spectacle lens 50 are changed according to the distances to the visual vision 44A, 44B and the like.
Thus, if the simulation image is displayed to correspond only to the movement of the head as described in Patent Document 1, when the visual line passing point is fixed, the vision will be largely different from the actual vision.
In the present embodiment, the system shown in FIG. 1 is used to display the simulation image also corresponding to the change of the positions of the visual line passing point 46A, 46B on the spectacle lens 50.
Next, the steps until the simulation image is displayed will be described with reference to a flowchart of FIG. 3.
The simulation steps performed by the system shown in FIG. 1 will be described below with reference to FIG. 3.
First, the position of the face, the direction (the direction of the head 42), and the visual line direction of the spectacle wearer 42 are detected by the gyro sensor (i.e., the tracking sensor for head movement 12) and the visual line tracking device (i.e., the tracking sensor for visual line 13) mounted on the HMD 11. The detection for the right eye is performed in Step S1, and the detection for the left eye is performed in Step S2.

On the other hand, in Step S3, the CG imaginary objects (i.e., the three-dimensional CG model) shown in FIG. 2 are prepared.

Next, in Step S4, by using a CG walk-through function, a visual vision cut off from the three-dimensional CG model is obtained according to the position of the face, the direction, and the visual line direction detected in Step S1 and Step S2. The visual vision has different visual fields respectively for the right eye and the left eye.

Next, in Step S5, an original image with no distortion and blur for the right eye is created based on the visual vision cut off in Step S4. Similarly, in Step S6, an original image with no distortion and blur for the left eye is created based on the visual vision cut off in Step S4.

On the other hand, in Step S7, the prescribed power, the addition power of the spectacle wearer 41 and the lens kind are inputted by the input device (such as the keyboard 19 or the like).

Next, based on the inputted content, shape data and layout data of the right lens are created in Step S8, and shape data and layout data of the left lens are created in Step S9.

Further, based on the inputted content, a right eyeball model is created in Step S10, and a left eyeball model is created in Step S11.

Next, based on the shape data, the layout data and the eyeball models respectively created in Steps S8 to S10, a three-dimensional spline interpolation coefficient of the light-ray data is generated in Step S12.

Next, in Step S13, the three-dimensional spline interpolation coefficients of various parameters such as outgoing light-ray direction, PSF parameter, lens passing point position and the like for the right eye are obtained by using the three-dimensional spline interpolation coefficient of the light-ray data generated in Step S12. Similarly, in Step S14, the three-dimensional spline interpolation coefficients of various parameters such as outgoing light-ray direction, PSF parameter, lens passing point position and the like for the left eye are obtained.

Next, in Step S15, the simulation is performed using the original images created in Step S5 and Step S6, and the parameters and interpolation coefficients obtained in Step S13 and Step S14. The processing of the simulation includes use of image processing hardware.

Next, in Step S16, an image containing blur and distortion for the right eye is created. Similarly, in Step S17, an image containing blur and distortion for the left eye is created.

The image containing blur and distortion created in the aforesaid manner is displayed on the display screen of the HMD 11, the monitor 16 for monitoring the right eye, and the monitor 17 for monitoring the left eye.

By the aforesaid process, the image containing blur and distortion corresponding to the visual line direction is displayed on the display screen of the HMD 11.

<2. Description of theory and method of moving image simulation>

2-1. Purpose of simulation

The purpose of the moving image simulation is to express how the spectacle wearer will see if he (or she) has worn spectacles by using a still image and a moving image.

By using a combination of the three-dimensional CG and the HMD 11, the gyro sensor and the visual line tracking device, it is possible to present an image seen when turning head and/or changing visual line in a virtual space on a real-time basis.

Further, as shown in the system of FIG. 1 and the flowchart of FIG. 3, it is possible to provide a binocular stereo vision if different images are respectively presented to the right and left eyes.

2-2. Coordinate system used in simulation

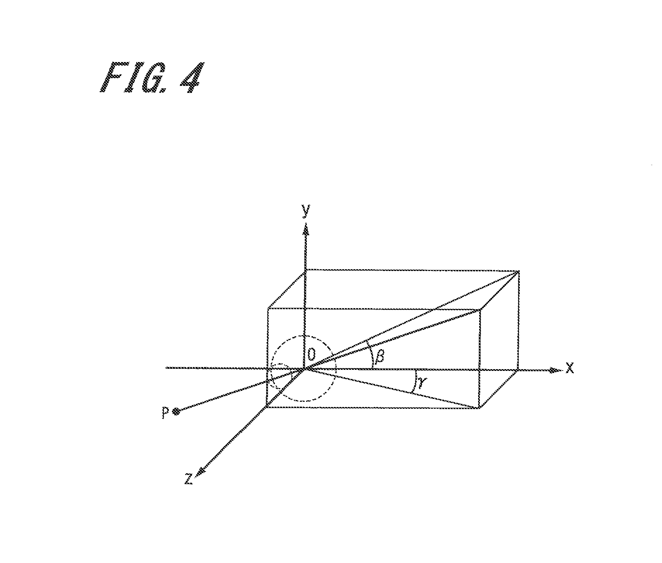

The coordinate system used in the simulation according to the present embodiment is shown in FIG. 4.

A coordinate system having an X-axis, a Y-axis, and a Z-axis is defined as shown in FIG. 4. The X-axis is defined as a direction extending from the front side to the eye. The Y-axis is defined as an upward direction perpendicular to the X-axis. The Z-axis is defined as a horizontal direction extending from the right to the left. The directions of the X-axis, the Y-axis, and the Z-axis are defined by the right-hand rule. Further, the origin is located at the center of cycloduction. The broken line in the drawing schematically shows the eyeball and the cornea.

Further, in the coordinate system shown in FIG. 4, an arbitrary point P (x, y, z) (herein x<0, which means the point P is located before the eye) can be expressed by angles $\beta$, $\gamma$ of the light-ray entered into the eye and a distance PO between the point P and the center of cycloduction.

The position in the simulation image is: $\tan\beta=y/x$ in the longitudinal direction, and $\tan\gamma=z/x$ in the lateral direction. In the case of spectacles, it is convenient to express the distance of the object by the inverse of the distance, rather than by the distance as it is. Thus, the position of an arbitrary point in the space can be expressed as follows.

$$D_l = 1/\sqrt{x^2+y^2+z^2} \,,$$

$$\psi = \tan\beta = y/x \,,$$

$$\zeta = \tan\gamma = z/x$$

2-3. Description of distortion of lens

The light-ray will be inflected when being seen through a lens.

In other words, the object point located in a direction $(\psi, \zeta)$ seen by a naked eye will move to $(\psi', \zeta')$ when seen through a spectacle lens.

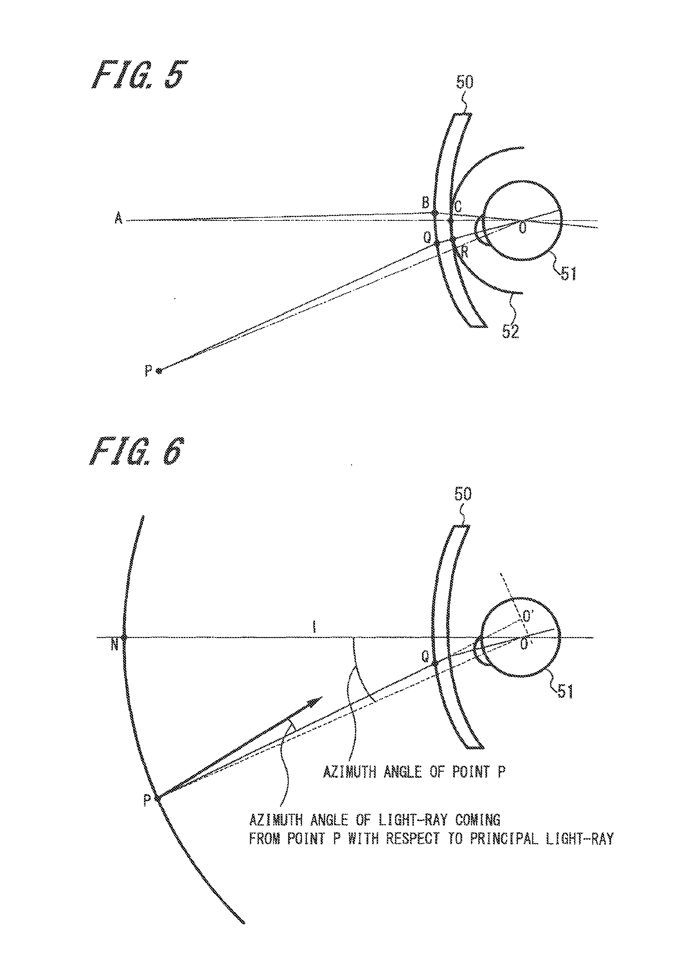

Thus phenomenon will be described in more detail with reference to FIG. 5. FIG. 5 shows the spectacle lens 50, an eyeball 51, and a back vertex sphere 52 corresponding to the spectacle lens 50.

With respect to an arbitrary point P shown in FIG. 5, the incident direction in the case of the naked eye is PO, while when wearing the spectacles to look through the spectacle lens 50, the incident direction to the center of cycloduction O of the eyeball 51 will be changed into RO.

Similarly, with respect to a point A shown in FIG. 5, the incident direction in the case of the naked eye is AO, while when looking through the spectacle lens 50, the incident direction to the center of cycloduction O of the eyeball 51 will be changed into BO.

Here, the position $(\psi', \zeta')$ of the object felt when wearing the spectacles can be expressed by a function of the position $(D_1, \psi, \zeta)$ felt in the case of the naked eye. To be specific, the position $(\psi', \zeta')$ of the object felt when wearing the spectacles can be expressed by the following function.

$$\psi' = \psi'(D_1, \psi, \zeta)$$

$$\zeta' = \zeta'(D_1, \psi, \zeta)$$

The content of the above function can be determined by light-ray tracing (which is to be described later).

2-3. Description of blur of lens

The reason of the blur caused by the lens is because not all light-rays from the object point are converged on one point of the retina.

The light from the object point forms a light distribution which spreads to a certain range with an image point as the center. Such a distribution is called PSF (point spread function).

A method for obtaining the PSF will be described below with reference to FIG. 6.

To obtain the PSF, a principal light-ray PQO passing through the point P is searched firstly.

After the principal light-ray has been determined, an entrance pupil is equally-divided (into 400 divisions, for example), the light-rays connecting the point P with each of divided areas are traced, and the points interacted with the retina are obtained.

In FIG. 6, each light-ray can be traced using the azimuth angle of the point P, and the azimuth angle of the light-ray coming from the point P with respect to the principal light-ray.

The position of the entrance pupil is, in a strict sense, a point conjugate to the object side of the pupil; however it is convenient to set the position of the entrance pupil as a point 0' in an extension of the line PQ, which is a portion of the principal light-ray on the object side, wherein the point 0' satisfies the condition of: PO=PO'.

If the PDF is based on the geometric optics principle, the density of the intersection points on the retina will be, in its entirety, the PSF.

In the case where the effect of wave optics is considered, the optical path difference between the light-rays of respective divided areas should be calculated, and Fresnel integral should be performed to obtain the PSF.

Next, various methods are considered as the method for dividing the entrance pupil.

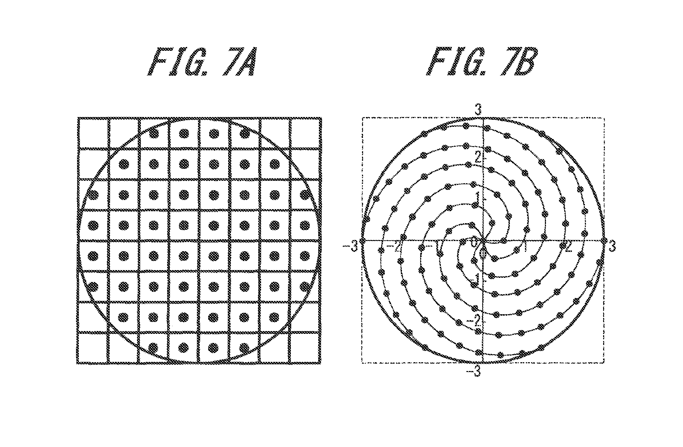

As main division methods, there are two kinds of division methods which are a tetragonal division shown in FIG. 7A and a spiral division shown in FIG. 7B.

In the tetragonal division shown in FIG. 7A, the area is divided vertically and horizontally, and the center point of each divided area is used. In such a case, it is only possible to trace about 70 percent of the coming light-rays simply because of the existence of useless parts at four corners.

While in the spiral division shown in FIG. 7B, points in the curves spirally extending from the center point of the entrance pupil are used. In such a case, it is possible to trace all coming light-rays.

Incidentally, it is preferred that the spiral arrangement includes at least two spirals. By including at least two spirals, it is possible to more efficiently use the entrance pupil compared with the case where only one spiral is included.

Further, in the case where six spirals are included as shown in FIG. 7B, it is possible to most efficiently use the entrance pupil.

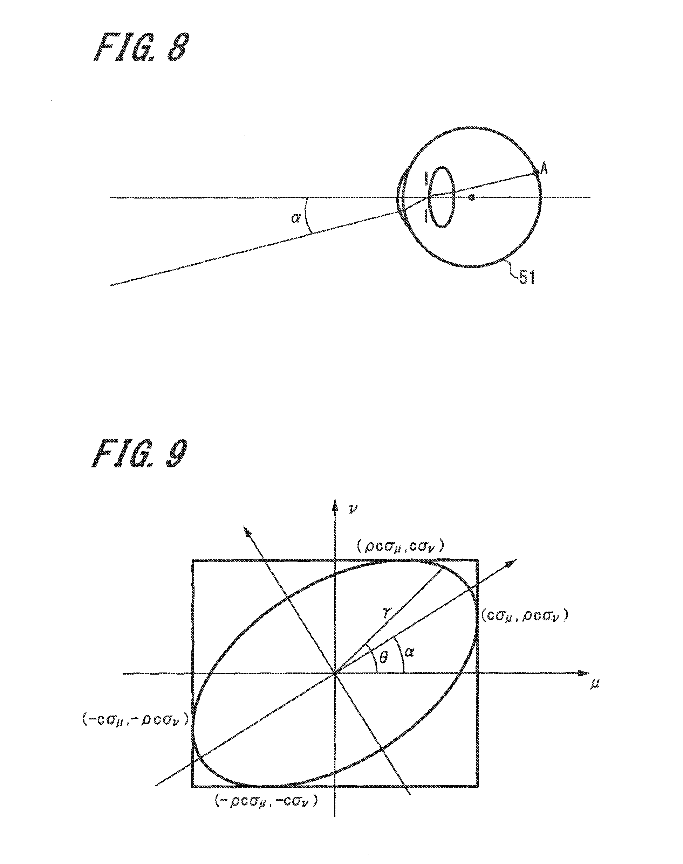

The PSF obtained in the aforesaid manner represents the light density distribution on the retina. However, since the coordinate of the incident image is the coordinate ($\psi$ $\zeta$) in the direction seen from the center of cycloduction, it is necessary to perform a conversion between the coordinate on the retina and the coordinate ($\psi$, $\zeta$) of the incident image. Here, the relationship between the incidence angle and the image height is shown in FIG. 8. It is considered that, in the effective range of the PSF, the image height is small, and sufficiently high accuracy can be obtained in paraxial calculation. To be specific, $\psi_m = y_m/f$, and $\zeta_m = z_m/f$. Here, f represents the focal length of the eye, and f varies according to the prescribed power.

Thus, the light distribution in the position of the retina can be converted into the light distribution in the direction of the incident light-ray. In other words, the eye feels that the light from the object point is coming from a space in a certain range with the object point as the center, instead of only coming from the object point.

Further, there is influence between adjacent points, so that difference is not easy to distinct, and therefore blur is caused.

It is obvious that the PSF will be different if looking through the different position of the lens.

Further, even when looking through the same position of the lens, the PSF will be different if the object distance is different. Furthermore, even when looking at a point of the same object distance through the same position of the lens, the PSF will be different if the accommodation state of the eye is different.

2-5. Synthesis of simulation image

The distortion and the blur caused by the lens have been described as above.

It is possible to simulate the image seen when wearing a spectacle lens if the distortion and the blur are synthesized by using an image processing method. Further, it is possible to not only simulate a still image, but also simulate a moving image.

Concerning the distortion, the image processing with respect to the distortion can be achieved by obtaining the object side points corresponding to all pixels in the image side visual field, and applying the obtained result to the brightness information of the original image.

Concerning the blur, the image processing with respect to the blur can be achieved by "distributing" the brightness of each pixel to the peripheral pixels according to the PSF to reconstruct the brightness of all pixels of the image.

The blur processing is also called "convolution". The convolution herein differs from a general convolution in that the PSF is indefinite.

2-6. Coordinate transformation in a case where central visual line of visual field is designated

As described above, it is possible to simulate the entire visual field in a coordinate system in which the X-axis is defined as the direction extending from the front side to the eye (such a coordinate system is referred to as a "global coordinate system" hereinafter) by using the distortion information (the conversion from an object-side vision direction to an image-side vision direction) and the blur information (the PSF in specified vision direction and visual distance).

However, in the actual simulation, the central visual line does not have to be directed to the front side.

For example, in the case where the vision of the near portion wants to be confirmed, the central visual line needs to pass through the near portion of the lens. In such case, the central visual line is in an oblique direction of the global coordinate system. The simulation is performed in a local coordinate system whose X'-axis is defined as the oblique direction.

At this time, the problem is how to determine the Y'-axis and Z'-axis of the local coordinate system. Herein, the Y'-axis and Z'-axis of the local coordinate system is determined according to the Listing's law, which is one of the laws regarding cycloduction. According to the Listing's law, when the central visual line is directed to the front side, the up-down direction and the right-left direction respectively change in a given direction corresponding to the movement of the central visual line caused by cycloduction. Also, when the central visual line changes and thereby the visual line direction changes, the up-down direction and the right-left direction of the actual object change so that the up-down direction and the right-left direction of the actual object also be the up-down direction and the right-left direction in the retinal image.

The coordinate axis transform matrix is shown as the following equation (2)

$$Q = \begin{bmatrix} a & b & c \\ -b & 1-\dfrac{b^2}{1+a} & -\dfrac{bc}{1+a} \\ -c & -\dfrac{bc}{1+a} & 1-\dfrac{c^2}{1+a} \end{bmatrix} \quad \text{------ (2)}$$

(here, a, b, c represent respective axial constituents of the directional unit vector (a b c) in the global coordinate system of the central visual line direction)

Further, the local coordinate (x', y', z') of an arbitrary point (x, y, z) of the global coordinate is converted according to the following equation (3).

$$\begin{pmatrix} x' \\ y' \\ z' \end{pmatrix} = Q \begin{pmatrix} x \\ y \\ z \end{pmatrix} = \begin{bmatrix} a & b & c \\ -b & 1-\dfrac{b^2}{1+a} & -\dfrac{bc}{1+a} \\ -c & -\dfrac{bc}{1+a} & 1-\dfrac{c^2}{1+a} \end{bmatrix} \begin{pmatrix} x \\ y \\ z \end{pmatrix} \quad \text{------ (3)}$$

In contrast, the global coordinate (x, y, z) of an arbitrary point (x', y', z') of the local coordinate is converted according to the following equation (4).

$$\begin{pmatrix} x \\ y \\ z \end{pmatrix} = Q^T \begin{pmatrix} x' \\ y' \\ z' \end{pmatrix} = \begin{bmatrix} a & -b & -c \\ b & 1-\dfrac{b^2}{1+a} & -\dfrac{bc}{1+a} \\ c & -\dfrac{bc}{1+a} & 1-\dfrac{c^2}{1+a} \end{bmatrix} \begin{pmatrix} x \\ y \\ z \end{pmatrix} \quad \text{------ (4)}$$

By using the aforesaid coordinate transformation equations, it is possible to really simulate the distortion in the case where the central visual line is the visual line direction passing through an arbitrary point on the lens.

2-7. Spline interpolation approximation of light-ray data

The optical principle and image processing method of simulating the vision seen through the spectacle lens have been established as above.

However, when actually performing simulation, vast calculations will be an annoying problem. The shape of the spectacle lens is not limited to a simple spherical surface. Particularly, in a progressive-addition lens, the shape of the spectacle lens is a free-form surface.

A repeat convergence method is employed to perform light-ray tracing of a complex surface such as the spectacle lens. The time necessary to perform such light-ray tracing is at least several times longer than the time necessary to perform light-ray tracing of a simple spherical surface.

Further, vast number of pixels of the simulation image is another factor associated with increased calculations.

The light-ray tracing for determining each pixel of the resultant image corresponds to which pixel of the original image has to be performed for all pixels of all resultant images of the simulation. Further, in order to determine the PSF, many light-rays (for example, 100 pieces) from the corresponding object point are traced to obtain the spots on the retina. Since all of these light-rays are traced with respect to an aspheric surface, the repeat convergence method has been employed, and therefore there will be tremendously vast calculations.

Given the calculation capability of one current personal computer, it will take several days to process one image (one frame of a moving image) by using such method.

On the other hand, with respect to an arbitrary point on the object side as follows, the image side $(\psi', \zeta)$ is uniquely determined under the condition that the lens shape, the eyeball parameter, and the position relation between the lens and the eyeball are all determined.

$$D_l = 1/\sqrt{x^2+y^2+z^2} \, ,$$

$$\psi = \tan\beta = y/x \, ,$$

$$\zeta = \tan\gamma = z/x$$

To be specific, the following functions are true.

$$\psi' = \psi'(D_1, \psi, \zeta)$$

$$\zeta' = \zeta'(D_1, \psi, \zeta)$$

Further, it is easy to imagine that functions $(\psi', \zeta')$ change continuously with respect to the variables $(D_1, \psi, \zeta)$. Such functions are suitable for performing spline interpolation.

Thus, a limited number of sample points are set within the domain of each variable. For example, fifteen points (-0.2, 0.0, 0.2, 0.5, 0.8, 1.1, 1.4, 1.7, 2.0, 2.3, 2.6, 2.9, 3.2, 3.6, 4.0) are set as the sample points of the object distance inverse $D_1$, fifteen points (-1.5, -1.2, -1.0, -0.8, -0.6, -0.4, -0.2, 0.0, 0.2, 0.4, 0.6, 0.8, 1.0, 1.2, 1.5) are set as the sample points of tangent $\psi$ of the up-down angle, and fifteen points (-1.5, -1.2, -1.0, -0.8, -0.6, -0.4, -0.2, 0.0, 0.2, 0.4, 0.6, 0.8, 1.0, 1.2, 1.5) are set as the sample points of tangent $\zeta$ of the right-left angle.

The light-ray tracing is performed on all combinations of these sample points to obtain true function values.

There are method of interpolating the function values of the other variable values (the values between sample points) using the true function values of the sample points. There are many interpolation methods corresponding to different purposes. B spline method is the most suitable interpolation method for the case where the true values of sample points are known.

Sample point number and sample point interval are related to interpolation accuracy. Generally, high interpolation accuracy can be achieved in the place where sample point interval is small. However, if the sample point interval is reduced, the sample point number will be increased to cover the whole domain, and therefore it is necessary to increase the program memory. Since a current PC and OS can be configured to have large memory, the limitation on the sample point number is relaxed, so that it has become possible to obtain high-accuracy results.

In such a manner, it becomes possible to obtain the following function (which expresses distortion information) by performing the spline interpolation with less calculations.

$$\psi' = \psi'(D_l, \psi, \zeta) = \sum_{i,j,k} C_{\psi i,j,k} N_k(\zeta) N_j(\psi) N_i(D_l)$$

$$\zeta' = \zeta'(D_l, \psi, \zeta) = \sum_{i,j,k} C_{\zeta i,j,k} N_k(\zeta) N_j(\psi) N_i(D_l)$$

Where C represents an interpolation coefficient, and N represents a basis polynomial function based on the node of each dimension.

2-8. Simplification of PSF

As describe above, in a strict sense, in order to obtain the PSF of a certain object point, the light-rays passing through many points that equally divide the entrance pupil are necessary to be traced to obtain the spots on the retina, and further, obtain a spots density distribution function.

However, with such method, the accuracy is not increased as much as expected even the number of the light-rays is increased.

Further, there is not only a case where, when the distortion is small, the spots concentrate, and the light-rays hardly pass through the places other than the image points, but also a case where, when the power error is large, the spots are evenly distributed in a certain area, so that the PSF function changes intensively.

On the other hand, in the case of performing simulation and/or performing lens performance evaluation, it is not necessary to have an accurate PSF. For example, eyesight represents the closest distance between two discriminable points (visual angle). In such a case, the precise form of the PSF function is not necessary, but the size of the range covering the PSF is an important parameter. Thus, the role in performing lens performance evaluation will not be significantly affected even if the PSF is boldly simplified.

In contrast, if previously assuming that the PSF is a continuous function and applying its parameters to the PSF by using the light-ray tracing data, the PSF can be expressed with less parameters. These parameters can be obtained by performing spline interpolation (three-dimensional), as the aforesaid distortion function.

To be able to approximate the PSF of the power error and astigmatism and all axis angles, it is appropriate that the form of the simplified function is a two-dimensional normal distribution. To be specific, it is appropriate that the simplified function is the following equation (5).

$$f(\mu, \nu) = \frac{1}{2\pi \sigma_\mu \sigma_\nu \sqrt{1-\rho^2}} \exp\left(-\frac{1}{2(1-\rho^2)}\left(\frac{\mu^2}{\sigma_\mu^2} - 2\rho \frac{\mu\nu}{\sigma_\mu\sigma_\nu} + \frac{\nu^2}{\sigma_\nu^2}\right)\right) \text{----- (5)}$$

(here, $\mu$, $\nu$ are respectively the deflection angles from the principal light-rays of Y and Z directions, and $\sigma_\mu$, $\sigma_\nu$, $\rho$ are parameters of the normal distribution. These parameters satisfy the conditions of: $\sigma_\mu > 0$, $\sigma_\nu > 0$, $-1 < \rho < 1$)

With respect to all points ($\mu$, $\nu$) in the line of the ellipse expressed by the following equation (6), the equation (1) mentioned above is true.

$$\frac{1}{2(1-\rho^2)}\left(\frac{\mu^2}{\sigma_\mu^2} - 2\rho \frac{\mu\nu}{\sigma_\mu\sigma_\nu} + \frac{\nu^2}{\sigma_\nu^2}\right) = \frac{c^2}{2} \qquad \text{------ (6)}$$

$$f(\mu, \nu) = \frac{1}{2\pi \sigma_\mu \sigma_\nu \sqrt{1-\rho^2}} \exp\left(-\frac{c^2}{2}\right) \quad \text{----- (1)}$$

Further, the integral within the contour ellipse is the following equation (7).

$$P(c) = \iint_c f(\mu, \nu) \, d\mu \, d\nu = 1 - \exp\left(-\frac{c^2}{2}\right) \qquad \text{------ (7)}$$

An equiprobability ellipse in such a case is shown in FIG. 9.

Thus, the two-dimensionalnormal distribution function can express the level of the spread ($\sigma_\mu$, $\sigma_\nu$), the level of the astigmatic blur (ratio of major axis to minor axis of the equiprobability ellipse), and the angle (angle of the major axis). Obviously, it is impossible to faithfully express an almost infinite change due to the state of the optical system of the PFS, however it is effective as a simplified function for expressing the PSF.

A considerable method of obtaining the parameters $\sigma_\mu$, $\sigma_\nu$, $\rho$ of the two-dimensional normal distribution function based on the light-ray data is the method of obtaining statistical values of the intersection points of many light-rays distributed on the plane ($\mu$, $\nu$) and applying the statistical values to $\sigma_\mu$, $\sigma_\nu$, $\rho$. To be specific:

$$\sigma_\mu = \sqrt{\frac{1}{N} \sum_i \mu_i^2}$$

$$\sigma_\nu = \sqrt{\frac{1}{N} \sum_i \nu_i^2}$$

$$\rho = \frac{1}{N} \sum_i \mu_i \nu_i \,/\, \sigma_\mu \sigma_\nu$$

Here, N represents the number of light-rays, and ($\mu_i$, $\nu_i$) represent the coordinates of the intersection points.

In such a manner, it is possible to approximate the PSF distribution function of an arbitrary point ($D_1$, $\psi$, $\zeta$) in the object space by the two-dimensional normal distribution function having the parameters $\sigma_\mu$, $\sigma_\nu$, $\rho$. Further, $\sigma_\mu$, $\sigma_\nu$, $\rho$ can be expressed as the functions of ($D_1$, $\psi$, $\zeta$) as follows.

$$\sigma_\mu = \sigma_\mu(D_1, \psi, \zeta)$$

$$\sigma_\nu = \sigma_\nu(D_1, \psi, \zeta)$$

$$P = \rho(D_1, \psi, \zeta)$$

Similar to the distortion information, theses functions can be obtained by performing spline interpolation. To be specific, theses functions can be obtained as follows.

$$\sigma_\mu = \sigma_\mu(D_l, \psi, \zeta) = \sum_{i,j,k} C_{\sigma\mu\,i,j,k} N_k(\zeta) N_j(\psi) N_i(D_l)$$

$$\sigma_\nu = \sigma_\nu(D_l, \psi, \zeta) = \sum_{i,j,k} C_{\sigma\nu\,i,j,k} N_k(\zeta) N_j(\psi) N_i(D_l)$$

$$\rho = \rho(D_l, \psi, \zeta) = \sum_{i,j,k} C_{\rho\,i,j,k} N_k(\zeta) N_j(\psi) N_i(D_l)$$

It should be noted that, due to spline interpolation error, there is a possible that the function values may go beyond the domain. For example, in the case where $\rho$ is $-1<\rho<1$, the result becomes $\rho=1.002$ if obtained by performing interpolation, which means the ellipse does not exist. An effective method to solve such a problem is to obtain $\sin^{-1} \rho$, instead of $\rho$, by performing interpolation, and perform a sine calculation on the result to obtain $\rho$.

In addition to the parameters of the distortion and blur, other useful parameters can be obtained by performing spline interpolation. Such parameters include, for example, lens convex surface passing point position ($y_{convex}$, $z_{convex}$) of the principal light-ray, lens concave surface passing point position ($y_{convex}$, $z_{convex}$) of the principal light-ray, and the like. Such parameters can be calculated as follows.

$$y_{convex} = y_{convex}(D_l, \psi, \zeta) = \sum_{i,j,k} C_{y_{convex}\,i,j,k} N_k(\zeta) N_j(\psi) N_i(D_l)$$

$$z_{convex} = z_{convex}(D_l, \psi, \zeta) = \sum_{i,j,k} C_{z_{convex}\,i,j,k} N_k(\zeta) N_j(\psi) N_i(D_l)$$

$$y_{concave} = y_{concave}(D_l, \psi, \zeta) = \sum_{i,j,k} C_{y_{concave}\,i,j,k} N_k(\zeta) N_j(\psi) N_i(D_l)$$

$$z_{concave} = z_{concave}(D_l, \psi, \zeta) = \sum_{i,j,k} C_{z_{concave}\,i,j,k} N_k(\zeta) N_j(\psi) N_i(D_l)$$

The lens passing point position of the principal light-ray is useful in performing simulation of the distribution of the distortion and blur in a local coordinate system whose central visual line is a transmitted light-ray at a particular position of the lens.

2-9. Speeding up multi-dimensional spline interpolation calculation

One-dimensional spline interpolation is expressed as follows.

$$F(x) = \sum_{i=1}^{n} C_i N_i(x)$$

Here, i represents the node number of each dimension, $C_i$ represents the coefficient thereof, and n represents the number of the sample points. $N_i(x)$ represents a basis function corresponding to i-th node. When the number of orders is M, $N_i(x)$ has a nonzero value in a range between i-th node and (i+M)-th node, and the internal between adjacent nodes is expressed by a (m-1)-order polynomial (due to the partiality of the basis function).

In other words, at an arbitrary point a within the domain of x, there only exists at most M pieces of nonzero $N_i(x)$.

Thus, it seems like there are n terms when looking the interpolation equation, however there actually are M terms at x=a, and F(a) can be obtained by performing M multiplications and M additions.

Three-dimensional spline interpolation is expressed by the following equation (8).

$$F(D_l, \psi, \zeta) = \sum_{i,j,k} C_{i,j,k} N_k(\zeta) N_j(\psi) N_i(D_l) \quad \text{------ (8)}$$

Here, i, j, k represent the node numbers of each dimension, and respectively change by the numbers of the sample points. In other words, the number of the terms is equal to the product of the number of the sample points of each dimension. However, due to the partiality of the aforesaid basis function, the number of the nonzero terms at a certain point is equal to the product of the number of the orders of each dimension.

In the case where the number of the orders of the spline of each dimension is 4, the number of the terms is $4^3$=64. In other words, the number of the calculations of one interpolation is 64 additions and $64\times3$=192 multiplications.

Generally, the number of necessary multiplications for performing an nj-dimensional M-order spline interpolation calculation is $nj\times M^{nj}$, and therefore if the number of dimensions becomes large, the calculation burden will rapidly increase,

However, if the above equation (8) is rewritten into the following equation (9), the number of calculation can be slightly reduced.

$$F(D_l, \psi, \zeta) = \sum_i \left( \sum_j \left( \sum_k (C_{i,j,k}) N_k(\zeta) \right) N_j(\psi) \right) N_i(D_l) \quad \text{------ (9)}$$

The equation (9) is a nested structure of a one-dimensional interpolation, and dimension order can be changed at will. The number of additions and the number of multiplications are both equal to $4+4\times(4+4\times4)$=84, so that the time necessary to perform calculation is reduced to 1/2.

Generally, the number of necessary multiplications for performing an nj-dimensional M-order spline interpolation calculation is expressed as following equation (10).

$$\sum_{i=1}^{nj} M^i = \frac{M}{M-1}(M^{nj}-1) \quad \text{------ (10)}$$

2-10. Summary

The moving image simulation using the three-dimensional CG has been described mainly focusing on the technical aspect. Since the three-dimensional outside world seen when wearing a progressive-addition lens is simulated by the moving image, the calculation amount is vast.

The vast amount of calculations can be reduced to a feasible level by performing spline interpolation and parameterizing the PSF.

Further, by applying the present invention, the necessary calculation time of the simulation can be reduced to 1/100 to 1/000, compared with the calculation time of the case where the present invention is not applied.

Actually, a spline coefficient database of the distortion and blur parameters of both the right eye and the left eye is previously prepared to generate a simulation image using a high-performance personal computer and a graphic board.

Further, the simulation image can be generated at a speed of 10 frames/second when a real time walk-through is performed in the CG with an HMD having a gyro mounted thereon.

According to the aforesaid embodiment, in the processing of adding distortion, the object side points corresponding to all pixels in the image side visual field is obtained, and the obtained result is applied to the brightness information of the original image.

Further, according to the aforesaid embodiment, in the processing of adding blur, the brightness of each pixel is "distributed" to the peripheral pixels according to the PSF to reconstruct the brightness of all pixels of the image. Furthermore, the normal distribution function and the parameters expressed as equation (1) is used.

In the present invention, the method of adding blur and distortion is not limited to the method described in the aforesaid embodiment, but includes other methods.

Further, either or both of the processing of adding distortion and the processing of adding blur can be performed by other methods than the method described in the aforesaid embodiment.

Further, in the aforesaid embodiment, the image for right eye and the image for left eye are respectively created so that it is possible to obtain a stereoscopic vision.

However, the present invention also includes a configuration in which only the image for the right eye or the image for the left eye is created.

Further, the present invention is not limited to the configuration in which the image is displayed on both the HMD 11 and monitors 16, 17 as described in the aforesaid embodiment, but includes other configurations such as the one in which there is only one display section, such as the case where the monitors are omitted and the image is displayed only on the HMD or the like, and the retinal image is displayed on the display section.

In the present invention, the display section for displaying the retinal image is not limited to the HMD and the monitor. Further, the sensor for detecting the movement of the head is not limited to the gyro sensor.

Furthermore, the method for displaying visual field image of the present invention is not limited to performing processing of adding blur and distortion corresponding to the change in the visual line direction as described in the aforesaid embodiment, but can be applied to a wider range of visual field image display device for spectacles (display system).

For example, the present invention can also be applied to a device (system) configured to only perform either the processing of adding distortion or the processing of adding blur.

It should be understood that the present invention is not limited to the aforesaid embodiment, but includes various other configurations without departing from the spirit of the present invention.

**Explanation of Reference Numerals**

| | |
|---|---|
| 11 | HMD |
| 15 | PC |
| 16, 17 | monitor |
| 41 | spectacle wearer |
| 42 | head |
| 43A, 43B | principal visual line |
| 44A, 44B | visual vision |
| 46A, 46B | visual line passing point |
| 50 | spectacle lens |

**Claims**

1. A visual field image display device for spectacles adapted to display a retinal image seen when wearing a progressive-addition lens by simulation, the device comprising:

   an image processing section adapted to create the retinal image by performing a processing of adding blur and distortion to data of an original image in a visual field corresponding to the direction of a visual line of an eye of a person in a state where the person wears the progressive-addition lens, wherein the blur and distortion correspond to a passing point of the visual line on the progressive-addition lens; and
   a display section adapted to display the retinal image created by the image processing section.

2. The visual field image display device for spectacles according to claim 1, wherein the distortion is calculated by using brightness information of the original image, the brightness information being specified at a point in a visual field on an object side corresponding to a pixel sampled in a visual field on an image side.

3. The visual field image display device for spectacles according to claim 1 or 2, wherein the blur is calculated based on a continuous function which expresses a light distribution of light-rays from the object, the light-rays spreading with an image point as the center.

4. The visual field image display device for spectacles according to claim 3, wherein the processing of adding blur is performed by distributing the brightness of each pixel of the display section to peripheral pixels base on the continuous function, and recalculating the brightness of all pixels of the image.

5. The visual field image display device for spectacles according to claim 4, wherein the processing of adding blur is performed by using a normal distribution function and parameters expressed as the following equation (1) and including an equiprobability ellipse which expresses the level of the spreading, the distortion and the blur of the image point:

$$f(\mu, \nu) = \frac{1}{2\pi\sigma_\mu\sigma_\nu\sqrt{1-\rho^2}}\exp\left(-\frac{c^2}{2}\right) \quad \text{------ (1)}$$

(here, $\mu$, $\nu$ are respectively the deflection angles of the principal light-ray from the coordinate axis direction, and $\sigma_\mu$, $\sigma_\nu$, $\rho$ are parameters of the normal distribution, wherein $\sigma_\mu > 0$, $\sigma_\nu > 0$, $-1 < \rho < 1$)

6. The visual field image display device for spectacles according to claim 5, wherein the parameters are calculated based on a one-dimensional nested structure.

7. The visual field image display device for spectacles according to claim 1, wherein the direction of the visual line is identified by a visual line tracking device and a gyro sensor.

8. The visual field image display device for spectacles according to claim 7, wherein the display section includes a head mounted display, and the visual line tracking device and the gyro sensor are mounted on the head mounted display.

9. A method for displaying visual field image for spectacles adapted to display a retinal image seen when wearing a progressive-addition lens by simulation, the method comprising:

   calculating the retinal image to be displayed at each point of a point group spirally arranged within a visual field.

10. The method for displaying visual field image for spectacles according to claim 9, wherein the spiral arrangement includes at least two spirals.

11. The method for displaying visual field image for spectacles according to claim 10, wherein the spiral arrangement includes six spirals

FIG. 1

FIG. 2

EP 2 341 388 A1

# FIG. 3

**S1** — DETECT POSITION AND DIRECTION OF FACE, AND VISUAL LINE DIRECTION (FOR RIGHT EYE)

**S2** — DETECT POSITION AND DIRECTION OF FACE, AND VISUAL LINE DIRECTION (FOR LEFT EYE)

GYRO SENSOR, VISUAL LINE TRACKING DEVICE

**S3** — CG IMAGINARY OBJECTS

**S4** — CG WALK-THROUGH FUNCTION

**S5** — ORIGINAL IMAGE WITH NO DISTORTION AND BLUR (FOR RIGHT EYE)

**S6** — ORIGINAL IMAGE WITH NO DISTORTION AND BLUR (FOR LEFT EYE)

**S8** — SHAPE DATA AND LAYOUT DATA OF RIGHT LENS

**S9** — SHAPE DATA AND LAYOUT DATA OF LEFT LENS

**S7** — PRESCRIBED POWER, ADDITION POWER, AND LENS KIND

**S10** — RIGHT EYEBALL MODEL

**S11** — LEFT EYEBALL MODEL

**S12** — GENERATE THREE-DIMENSIONAL SPLINE INTERPOLATION COEFFICIENT OF LIGHT-RAY DATA

**S13** — THREE-DIMENSIONAL SPLINE INTERPOLATION COEFFICIENTS OF VARIOUS PARAMETERS SUCH AS OUTGOING LIGHT-RAY DIRECTION, PSF PARAMETER, LENS PASSING POINT POSITION AND THE LIKE FOR RIGHT EYE

**S14** — THREE-DIMENSIONAL SPLINE INTERPOLATION COEFFICIENTS OF VARIOUS PARAMETERS SUCH AS OUTGOING LIGHT-RAY DIRECTION, PSF PARAMETER, LENS PASSING POINT POSITION AND THE LIKE FOR LEFT EYE

**S15** — SIMULATION (INCLUDING IMAGE PROCESSING HARDWARE)

**S16** — IMAGE CONTAINING BLUR AND DISTORTION (FOR RIGHT EYE)

**S17** — IMAGE CONTAINING BLUR AND DISTORTION (FOR LEFT EYE)

MONITOR FOR MONITORIONG RIGHT EYE

MONITOR FOR MONITORIONG LEFT EYE

HMD

EP 2 341 388 A1

FIG. 4

# FIG. 5

# FIG. 6

AZIMUTH ANGLE OF POINT P

AZIMUTH ANGLE OF LIGHT-RAY COMING
FROM POINT P WITH RESPECT TO PRINCIPAL LIGHT-RAY

FIG. 7A

FIG. 7B

FIG. 8

FIG. 9

| INTERNATIONAL SEARCH REPORT | International application No. |
|---|---|
| | PCT/JP2009/067693 |

**A. CLASSIFICATION OF SUBJECT MATTER**
*G02C13/00*(2006.01)i, *G02C7/06*(2006.01)i

According to International Patent Classification (IPC) or to both national classification and IPC

**B. FIELDS SEARCHED**

Minimum documentation searched (classification system followed by classification symbols)
G02C13/00, G02C7/06

Documentation searched other than minimum documentation to the extent that such documents are included in the fields searched
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2002-45336 A (Hoya Corp.), | 1-6,9 |
| A | 12 February 2002 (12.02.2002), | 7,8,10,11 |
| | entire text; all drawings | |
| | & US 2001/0056338 A1    & EP 1158338 A2 | |
| | & DE 60117432 T       & AT 319119 T | |
| | & HK 1038073 A | |
| | | |
| A | JP 2000-47154 A (Seiko Epson Corp.), | 1-8 |
| | 18 February 2000 (18.02.2000), | |
| | (Family: none) | |
| | | |
| A | JP 2005-261690 A (Pentax Corp.), | 1-11 |
| | 29 September 2005 (29.09.2005), | |
| | (Family: none) | |

☐ Further documents are listed in the continuation of Box C.     ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 30 November, 2009 (30.11.09) | 08 December, 2009 (08.12.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**REFERENCES CITED IN THE DESCRIPTION**

**Patent documents cited in the description**

- JP 3893760 B